# EUROPÄISCHE PATENTSCHRIFT

(11) **EP 2 842 543 B1**
(45) Veröffentlichungstag und Bekanntmachung des Hinweises auf die Patenterteilung: **21.02.2018**
(21) Anmeldenummer: 14002937.2
(22) Anmeldetag: 25.08.2014
(51) Int. Cl.: A61K 8/49, A61Q 19/00, A61K 8/97

(54) **Zusammensetzung mit Kühlwirkung für die topische Anwendung**
Composition with cooling effect for topical application
Composition avec effet réfrigérant pour application topique

(30) Priorität: 23.08.2013 DE 102013013986
(43) Veröffentlichungstag der Anmeldung: 04.03.2015
(73) Patentinhaber: S & V Technologies GmbH, 16761 Hennigsdorf (DE)
(72) Erfinder: Munteanu-Kempa, Maricica, D-13405 Berlin (DE); Choi, SooWhan, D-10247 Berlin (DE)
(74) Vertreter: Seuss, Thomas

(56) Entgegenhaltungen:
- CN-A- 101 288 754
- CN-A- 101 342 254
- CN-A- 102 343 096
- KR-A- 20050 067 951

## Beschreibung

Die Erfindung betrifft die nicht-therapeutische Verwendung einer topisch anwendbaren Zusammensetzung, die einen Kühleffekt hervorruft wie in Anspruch 1 beschrieben. Die Zusammensetzung ist insbesondere charakterisiert durch einen Anteil von Andrographolide. Die Zusammensetzung kann nach einer dermatologischen Behandlung verwendet werden um eine Kühlwirkung zu vermitteln. Sie kann aber auch zu rein kosmetischen Zwecken verwendet werden.

### Hintergrund der Erfindung

Bei einer Reihe von Eingriffen im Bereich der ästhetischen Chirurgie oder anderen dermatologischen Behandlungen treten Spannungsgefühle und Schmerzen im Bereich der behandelten Hautareale auf. Diese werden traditionellerweise durch Kühlung gelindert. Hierzu wird beispielsweise Wasser oder Eis verwendet. Diese Mittel wirken jedoch nur sehr kurzfristig. Andere Möglichkeiten zur Kühlung bestehen in der Verwendung verschiedener leichtflüchtiger Alkohole (Methanol oder Ethanol) oder aber durch Menthol bzw. Kampfer. Derartige Mittel sind jedoch insbesondere im Bereich des Gesichtes nicht anwendbar, da Sie reizend auf die Augen, aber auch auf Schleimhäute wirken. Vor diesem Hintergrund besteht ein Bedarf an Kühlmitteln, die dermatologisch verträglich sind, insbesondere keine Reizungen verursachen und eine langanhaltende Wirkung gewähren.
Es wurde nun gefunden, dass Zusammensetzungen enthaltend Andrographolide oder Derivate hiervon geeignet sind, nach topischer Applikation über einen längeren Zeitraum einen deutlichen Kühleffekt zu gewährleisten. Dabei wurden bislang keine Nebenwirkungen beobachtet. Der Kühleffekt hängt von der Konzentration ab.
Bei Anwendung der Zusammensetzung nach Beispiel 1 wird ein Kühleffekt beobachtet, der dem einer Mentholzusammensetzung entspricht und über mindestens 15-20 Minuten anhält.

Andrographolide ist ein Bestandteil der Pflanze *Andrographis paniculata,* welche auch als *"King of Bitters"* bekannt ist. Andrographis paniculata ist eine Pflanze, die insbesondere in Indien und Pakistan vorkommt und am besten unter tropischen Bedingungen gedeiht. Die Pflanze findet Verwendung in der traditionellen ayurvedischen Medizin. Auch in der thailändischen und chinesischen Medizin ist die Pflanze bekannt. Sie wird unter anderem zur Behandlung von Erkältungen, Fieber, Flatulenz und Durchfallerkrankungen verwendet. Aufgrund der vermuteten antiinflammatorischen Wirkung wurden verschiedentlich entzündungshemmende Zusammensetzungen vorgeschlagen. Ein topischer Kühleffekt der Pflanze oder ihrer Inhaltsstoffe wurde bislang nicht beschrieben.

Überraschend ist, dass der oben bereits beschriebene Kühleffekt umgehend nach topischer Applikation eintritt und über längere Zeit anhält. Obgleich die Wirkungsweise der Zusammensetzung nicht klar ist kann in jedem Fall festgestellt werden, dass die Wirkung zumindest nicht allein auf dem möglicherweise eintretenden entzündungshemmenden Effekt beruht, denn die Kühlwirkung tritt auch auf einer völlig unbehandelten Haut auf, die keinerlei Entzündungszeichen zeigt.

Eine erfindungsgemäße Zusammensetzung kann beispielsweise durch Lösung eines Extraktes aus Blättern der Pflanze Andrographis paniculata hergestellt werden. Hierbei wirkt bereits eine 0,01%ige Lösung des Pflanzenextraktes außerordentlich gut. Für die Herstellung der Lösung ist es empfehlenswert ein Cyclodextrin zuzusetzen, beispielsweise Methylcyclodextrin. Die Erfindung betrifft daher eine wässrige Lösung enthaltend 0,001 - 0,1% eines Extraktes aus Blättern von Andrographis paniculata, ferner enthaltend 0 - 1,0% Cyclodextrin, bevorzugt Methylcyclodextrin.

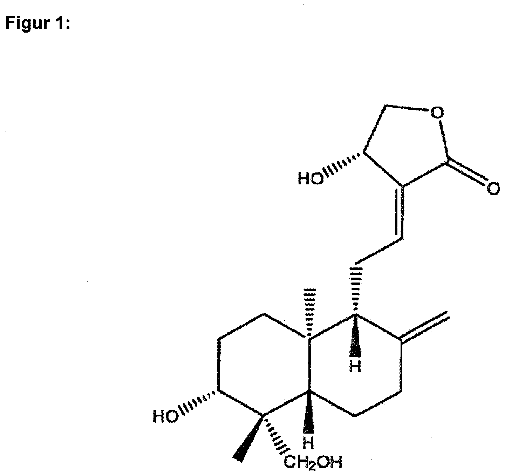

Es wurde gefunden, dass die erfindungsgemäße Wirkung insbesondere auf die Substanz Andrographolide (Figur 1) zurückgeht. Es versteht sich daher von selbst, dass auch isoliertes und gereinigtes Antrographolide oder synthetisches Andrographolide zur Erzielung einer erfindungsgemäßen Zusammensetzung verwendet werden kann. Da Andrographolide ca. 2% der Bestandteile der Blätter der Pflanze Andrographis paniculata darstellt sind entsprechend geringere Mengen der Reinsubstanz erforderlich, um die oben beschriebene Wirkung zu erzielen. Die erfindungsgemäße Wirkung tritt bei einer Andrographolide Konzentration von 0,01 - 1 % ein.

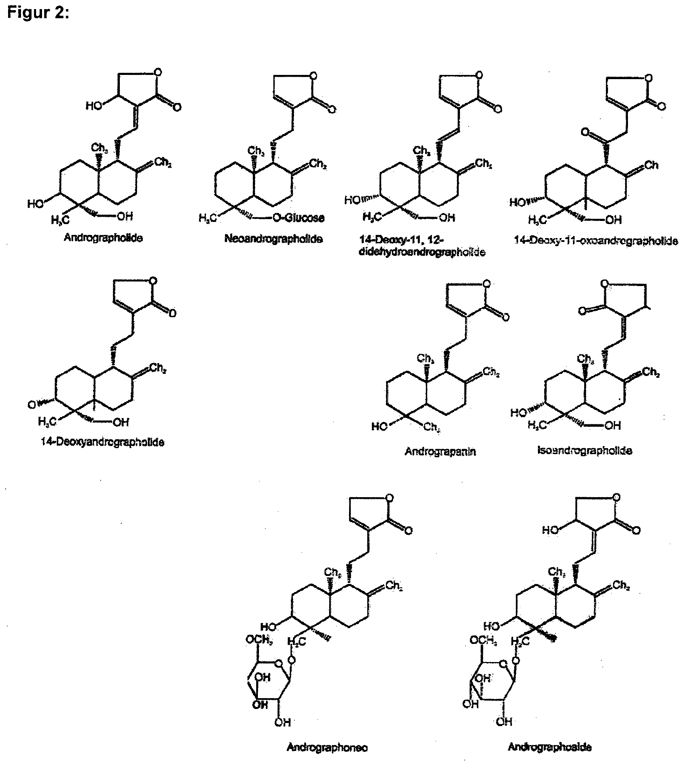

Es sind eine Reihe weiterer Verbindungen isoliert worden, welche ähnliche Strukturen und auch ähnliche Wirkungen zeigen, so dass auch diese vorteilhafterweise zur Erzielung des gewünschten Effekts verwendet werden können. Diese Verbindungen werden im Rahmen der vorliegenden Anmeldung als "Andrographolide-Derivate" bezeichnet und sind in der Figur 2 abgebildet. Die Erfindung betrifft daher auch Zusammensetzungen enthaltend ein oder mehrere Andrographolide Derivate gemäß Figur 2, nämlich Andrographolide, Neoandrographolide, 14-Deoxy-11,12-didehydroandrographolide, 14-Deoxy-11-oxoandrographolide, 14-Deoxyandrographolide, Andrograpanin, Isoandrographolide, Andrographoneo, Andrographoside. Da die kühlende Wirkung der Andrographolide Derivate leicht von der Wirkung von Andrographolide abweicht, sind ggf. kleinere Anpassungen der Konzentration nötig. Diese sind vom Fachmann auf dem Gebiet topischer Zusammensetzungen durch routinemäßiges Handeln leicht herstellbar, so dass die ggf. nötigen Tests einfach durchgeführt werden können. Die erfindungsgemäßen Zusammensetzungen sind daher charakterisiert durch einen Anteil von Andrographolide oder eines oder mehrerer Andrographolide Derivate, welche aus Pflanzen isoliert oder teil- oder vollsynthetisch hergestellt sein können. Die beschriebenen Zusammensetzungen können entweder reines Andrographolide oder ein einziges Andrographolide-Derivat enthalten, es können aber auch Mischungen dieser Komponenten verwendet werden.

Die erfindungsgemäße Zusammensetzung kann, über die bereits genannten Komponenten Wasser Andrographolide und/oder Andrographolide Derivate sowie ggf. Cyclodextrin, insbesondere Methylcyclodextrin, hinaus weitere Bestandteile enthalten, die die Stabilität der Zusammensetzung erhöhen, wie z.B. EDTA (z. B. als Di-Natriumsalz), Natriumtocopherylphosphat oder andere. Darüber hinaus können weitere Komponenten zugefügt werden, die ergänzende entzündungshemmende, abschwellende, pflegende oder sonstige Wirkungen begünstigen.

Die erfindungsgemäße Zusammensetzung kann insbesondere bei kosmetischen Operationen postoperativ verwendet werden, beispielsweise nach Hautstraffungen, Unterspritzungen oder ähnlichen Behandlungsmethoden. Aufgrund der guten, lang anhaltenden Wirkungen bei gleichzeitig hoher Verträglichkeit sind aber auch viele andere Einsatzgebiete möglich. So kann beispielsweise eine Kühlung nach kosmetischer Behandlung, w. z. B. Epilation (auch Laserepilation) vorgenommen werden. Weiterhin eröffnen sich Einsatzmöglichkeiten bei der therapeutischen und kosmetischen Behandlung von Sonnenbränden. Darüber hinaus kann beispielsweise auch nach Tätowierungen einer Kühlung durch die erfindungsgemäße Zusammensetzung erfolgen. Andrographolide und/oder Derivate davon können darüber hinaus Bestandteil kosmetischer Zubereitungen (z. B. Aftershavemitteln, Shampoos, Mundspülungen) sein. Weiterhin kann beispielsweise ein Einsatz in Form eines Duschgels erfolgen, der beispielsweise Sportlern einen Kühleffekt nach sportlicher Aktivität gewährleistet. Es besteht daher eine große Vielfalt von Einsatzmöglichkeiten der Erfindungsgemäßen Zusammensetzungen. Dabei ist es für den Fachmann selbstverständlich, dass ggf. eine Anpassung der Zusammensetzung erfolgen muss, wie z.B. durch eine Gelbildung oder eine Cremebildung.

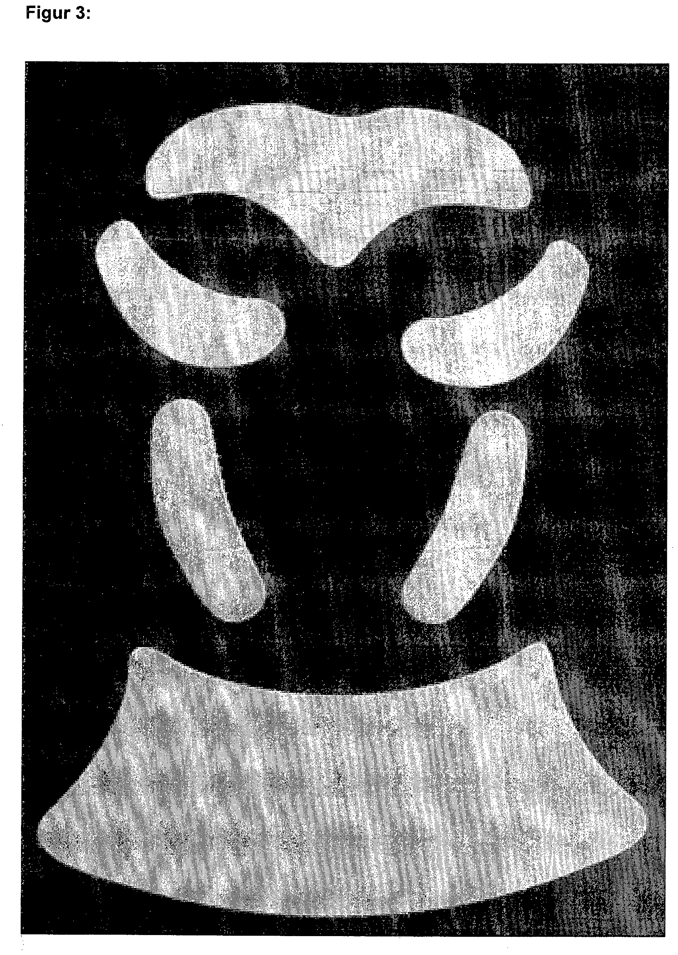

Eine besonders vorteilhafte Anwendung im Rahmen von kosmetischen Eingriffen besteht darin, dass Gewebestreifen (z. B. Baumwollgewebe) mit der erfindungsgemäßen Zusammensetzung getränkt und auf das behandelte Gewebe aufgelegt werden. So können beispielsweise derartige Baumwollgewebe auf das Gesicht nach einer kosmetischen Operation aufgelegt und somit die Kühlwirkung erzeugt werden. Dabei können entsprechende Gewebestreifen, die mit der erfindungsgemäßen Zusammensetzung getränkt sind bereits so zugeschnitten sein, dass sie das Gesicht optimal abdecken (siehe Figur 3). Diese Gewebestreifen können in einer sterilen Verpackung verschweißt zur Einmalverwendung vorbereitet sein und erlauben dem behandelnden Personal eine einfache Anwendung, durch Öffnen der Verpackung, Entnahmen der vorgeschnittenen Streifen und einfache Auflegung auf das Gesicht bzw. andere betroffene Körperteile.

Weitere Aspekte der Erfindung ergeben sich aus den Beispielen und benötigen an dieser Stelle keiner weiteren Vertiefung:

### Beispiel 1

Ein Bespiel einer erfindungsgemäßen Zusammensetzung hat die folgende Zusammensetzung

| | Bestandteil | % |
|---|---|---|
| A | Wasser (Aqua) | 98,74 |
| B | Methyl Cyclodextrin | 0,10 |
| C | Andrographis Paniculata Leaf Extract | 0,01 |
| D | Natriumtocopherylphosphat (Sodium Tocopheryl Phosphate) | 0,10 |
| E | Di-Natrium EDTA (Disodium EDTA) | 0,05 |
| F | Echinacea Angustifolia Extract | 1,00 |
| | Total | 100 |

Cyclodextrin wird in Wasser gelöst. Andrographis Paniculata Leaf Extract wird zugegeben und gerührt bis es sich komplett gelöst hat. Sodium Tocopheryl Phosphate wird zugegeben und gerührt. Di-Natrium EDTA wird unter Rühren in die Lösung eingegeben. Die Lösung wird gerührt bis sie klar ist. Danach wird Echinacea Angustifoliua Extract zugegeben und gerührt.

### Beispiel 1a

Eine Zusammensetzung wie in Beispiel 1 wird wie oben geschildert angerührt, wobei als Bestandteil C 0,01% reines Andrographolide verwendet wird.

### Beispiel 1b-q

Eine Zusammensetzung wie in Beispiel 1 wird wie oben geschildert angerührt, wobei als Bestandteil C
b) 0,01% reines Neoandrographolide,
c) 0,01% reines 14-Deoxy-11,12-didehydroandrographolide,
d) 0,01% reines 14-Deoxy-11-oxoandrographolide,
e) 0,01% reines 14-Deoxyandrographolide,
f) 0,01% reines Andrograpanin,
g) 0,01% reines Isoandrographolide,
h) 0,01% reines Andrographoneo,
i) 0,01% reines Andrographoside
j) 0,05% reines Neoandrographolide,
k) 0,05% reines 14-Deoxy-11,12-didehydroandrographolide,
l) 0,05% reines 14-Deoxy-11-oxoandrographolide,
m) 0,05% reines 14-Deoxyandrographolide,
n) 0,05% reines Andrograpanin,
ö) 0,05% reines Isoandrographolide,
p) 0,05% reines Andrographoneo,
q) 0,05% reines Andrographoside
r) 0,05% reines Andrographolide
verwendet wird. Bei den Beispielen 1j-1r wird die Wassermenge (Komponente A) entsprechend verringert, nämlich auf 98,70%.

### Beispiel 1s

Eine Zusammensetzung wie in Beispiel 1 wird wie oben geschildert angerührt, wobei als Bestandteil C Mischungen von zwei oder drei Verbindungen aus der folgenden Liste verwendet wird:
Andrographolide, Neoandrographolide, 14-Deoxy-11,12-didehydroandrographolide, 14-Deoxy-11-oxoandrographolide, 14-Deoxyandrographolide, Andrograpanin, Isoandrographolide, Andrographoneo, Andrographoside.

Der Gesamtgehalt der genannten Verbindungen wird als Komponente C kann 0,001 - 0,1% betragen, in der Regel werden 0,01-0,05% eingestellt. Die Wassermenge (Komponente A) wird entsprechen angepasst.

### Beispiel 2

Wenige Milliliter der erfindungsgemäßen Zusammensetzung nach Beispiel 1 werden mit einem Schwamm auf die Gesichtshaut aufgetragen. Der Proband verspürt über 15 - 20 Minuten einen deutlichen Kühleffekt.
Nebenwirkungen, insbesondere Reizungen der Augen und/oder Schleimhäute werden nicht beobachtet.

### Beispiel 2a

Analog zu Beispiel 2 werden Zusammensetzungen gemäß der Beispiele 1a-1s angewendet, wobei vergleichbare Wirkungen erzielt werden. Bei höheren Konzentrationen von Andrographolide bzw. von Andrographolide-Derivaten werden im Allgemeinen auch höhere Wirkungen (höhere Kühlwirkung, z.T. auch länger anhaltend) erzielt Auch hierbei werden keinerlei Nebenwirkungen festgestellt, auch nicht bei höheren Konzentrationen der Wirkstoffe.

### Beispiel 3

Gewebestreifen aus Baumwollgebe werden mit der erfindungsgemäßen Zusammensetzung nach Beispiel 1 getränkt und steril verpackt. Die Gewebestreifen können verschiedenste Größen und Formen aufweisen, so dass Sie an beliebigen Körperstellen verwendbar sind. Die Gewebestreifen sind über mindestens 12 Monate lagerfähig. Vor oder während eines dermatologischen Eingriffes wird die Verpackung geöffnet, die Gewebestreifen entnommen und unmittelbar auf das gewünschte Hautareal aufgetragen. Der Proband verspürt unmittelbar einen deutlichen Kühleffekt, der auch nach Entfernen der Gewebestreifen anhält. Nebenwirkungen, insbesondere Reizungen der Augen und/oder Schleimhäute werden nicht beobachtet.

Ein Beispiel eines Sets derartiger Gewebestreifen ist in der Figur 3 dargestellt: Von oben nach unten zu sehen sind Gewebestreifen für den Glabella-, Augen- (je 2 mal), Nasolabial- (je 2 mal), und Hals- Bereich.

## Patentansprüche

1. Nicht-therapeutische Verwendung einer Zusammensetzung enthaltend 0,01 - 1% Andrographolide,
eines Andrographolide Derivates, ausgewählt aus Neoandrographolide, 14-Deoxy-11,12-didehydroandrographolide, 14-Deoxy-11-oxoandrographolide, 14-Deoxyandrographolide, Andrograpanin, Isoandrographolide, Andrographoneo, Andrographoside oder Mischungen hiervon
zur topischen Anwendung zur Erreichung eines Kühleffektes auf unbehandelter Haut, die keinerlei Entzündungszeichen aufweist.

2. Nicht-therapeutische Verwendung der Zusammensetzung gemäß Anspruch 1, zusätzlich enthaltend 0,01 - 1,0% Cyclodextrin.

3. Nicht-therapeutische Verwendung der Zusammensetzung gemäß Anspruch 2, **dadurch gekennzeichnet, dass** das Cyclodextrin Methylcyclodextrin ist oder enthält.

4. Nicht-therapeutische Verwendung der Zusammensetzung gemäß mindestens eines der Ansprüche 1 bis 3, zusätzlich enthaltend 0,0 - 1,0% Natriumtocopherylphosphat.

5. Nicht-therapeutische Verwendung der Zusammensetzung gemäß mindestens eines der Ansprüche 1 bis 4, zusätzlich enthaltend 0,0 - 0,2% Di Natrium-EDTA.

6. Nicht-therapeutische Verwendung der Zusammensetzung gemäß mindestens einem der Ansprüche 1 bis 5 vor, während oder nach einer Epilation oder einer Tätowierung.

7. Nicht-therapeutische Verwendung eines gebrauchsfertigen Baumwollgewebestreifens, getränkt mit einer Zusammensetzung enthaltend 0,01 - 1% Andrographolide,
eines Andrographolide Derivates, ausgewählt aus Neoandrographolide, 14-Deoxy-11,12-didehydroandrographolide, 14-Deoxy-11-oxoandrographolide, 14-Deoxyandrographolide, Andrograpanin, lsoandrographolide, Andrographoneo, Andrographoside oder Mischungen hiervon
zur topischen Anwendung zur Erreichung eines Kühleffektes auf unbehandelter Haut, die keinerlei Entzündungszeichen aufweist.

8. Nicht-therapeutische Verwendung eines steril verpackten Baumwollgewebestreifens gemäß Anspruch 7.

## Claims

1. Non-therapeutic use of a composition containing 0.01 - 1 % of andrographolide,
of an andrographolide derivative, selected from neoandrographolide, 14-deoxy-11,12-didehydroan-drographolide, 14-deoxy-11-oxoandrographolide, 14-deoxyandrographolide, andrograpanine, isoandrographolide, andrographoneo, andrographoside, or mixtures thereof,
for topical application for obtaining a cooling effect on untreated skin not showing any signs of inflammation.

2. Non-therapeutic use of the composition according to claim 1, further containing 0.01 - 1.0 % of cyclodextrin.

3. Non-therapeutic use of the composition according to claim 2, **characterized by** that the cyclodextrin is or contains methyl cyclodextrin.

4. Non-therapeutic use of the composition according to at least one of claims 1 to 3, further containing 0.0 - 1.0 % of sodium tocopheryl phosphate.

5. Non-therapeutic use of the composition according to at least one of claims 1 to 4, further containing 0.0 - 0.2 % of disodium EDTA.

6. Non-therapeutic use of the composition according to at least one of claims 1 to 5 before, during, or after an epilation or a tattooing process.

7. Non-therapeutic use of a ready-to-use cotton cloth strip, soaked in a composition containing 0.01 - 1 % of andrographolide,
of an andrographolide derivative, selected from neoandrographolide, 14-deoxy-11,12-didehydroan-drographolide, 14-deoxy-11-oxoandrographolide, 14-deoxyandrographolide, andrograpanine, isoandrographolide, andrographoneo, andrographoside, or mixtures thereof,
for topical application for obtaining a cooling effect on untreated skin not showing any signs of inflammation.

8. Non-therapeutic use of a sterile packaged cotton cloth strip according to claim 7.

## Revendications

1. Utilisation non-thérapeutique d'une composition comprenant de 0,01 à 1 % d'andrographolide,
d'un dérivé d'andrographolide, choisi à partir de néoandrographolide, 14-déoxy-11,12-didehydroandrographolide, 14-déoxy-ll-oxoandrographolide, 14-déoxyandrographolide, andrograpanine, isoandrographolide, andrographonéo, andrographoside, ou mélanges de ceux-ci,
à application topique pour réaliser un effet de refroidissement sur la peau non-traitée ne comportant aucun symptôme d'inflammation.

2. Utilisation non-thérapeutique de la composition selon la revendication 1, en outre comprenant de 0,01 à 1,0 % de cyclodextrine.

3. Utilisation non-thérapeutique de la composition selon la revendication 2, **caractérisée en ce que** la cyclodextrine est ou comporte du méthyle cyclodextrine.

4. Utilisation non-thérapeutique de la composition selon au moins une des revendications 1 à 3, en outre comprenant de 0,0 à 1,0 % de tocophéryle phosphate de sodium.

5. Utilisation non-thérapeutique de la composition selon au moins une des revendications 1 à 4, en outre comprenant de 0,0 à 0,2 % de disodium EDTA.

6. Utilisation non-thérapeutique de la composition selon au moins une des revendications 1 à 5 avant, pendant ou après une épilation ou une procédure de tatouage.

7. Utilisation non-thérapeutique d'un ruban de tissu de coton prêt à l'emploi, imbibé d'une composition comprenant de 0,01 à 1 % d'andrographolide, d'un dérivé d'andrographolide, choisi à partir de néoandrographolide, 14-déoxy-11,12-didehydroandrographolide, 14-déoxy-11-oxoandrographolide, 14-déoxyandrographolide, andrograpanine, isoandrographolide, andrographonéo, andrographoside, ou mélanges de ceux-ci,
à application topique pour réaliser un effet de refroidissement sur la peau non-traitée ne comportant aucun symptôme d'inflammation.

8. Utilisation non-thérapeutique d'un ruban de tissu de coton selon la revendication 7 emballé de façon stérile.
